(19) Europäisches Patentamt
European Patent Office
Office européen des brevets

(11) EP 2 443 078 B1

(12) EUROPÄISCHE PATENTSCHRIFT

(45) Veröffentlichungstag und Bekanntmachung des Hinweises auf die Patenterteilung:
**04.04.2018 Patentblatt 2018/14**

(21) Anmeldenummer: **10725738.8**

(22) Anmeldetag: **18.06.2010**

(51) Int Cl.:
***C06B 23/00** (2006.01)* ***A01K 15/02** (2006.01)*
***C07C 407/00** (2006.01)*

(86) Internationale Anmeldenummer:
**PCT/EP2010/058660**

(87) Internationale Veröffentlichungsnummer:
**WO 2010/146170 (23.12.2010 Gazette 2010/51)**

(54) **DUFTPROBEN PEROXIDISCHER SPRENGSTOFFE**

ODOR SAMPLES OF PEROXIDIC EXPLOSIVES

ÉCHANTILLONS OLFACTIFS DE MATIÈRES EXPLOSIVES PÉROXYDIQUES

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **18.06.2009 DE 102009029787**

(43) Veröffentlichungstag der Anmeldung:
**25.04.2012 Patentblatt 2012/17**

(73) Patentinhaber:
- **Rheinische Friedrich-Wilhelms-Universität Bonn 53113 Bonn (DE)**
- **Diehl Defence GmbH & Co. KG 88662 ÜBERLINGEN (DE)**

(72) Erfinder:
- **WALDVOGEL, Siegfried 55218 Ingelheim (DE)**
- **SIERING, Carsten 55122 Mainz (DE)**
- **LUBCZYK, Daniel 55118 Mainz (DE)**
- **LÖBAU, Jörg 88633 Heiligenberg (DE)**
- **HAHMA, Arno Henfenfeld 91239 (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
**WO-A2-2004/035018**
**WO-A2-2007/005471**
**US-A- 3 538 011**
**US-A- 4 455 252**
**US-A- 5 648 636**
**US-A1- 2006 135 822**
**US-A1- 2006 183 654**

- **BAJ, STEFAN ET AL: "A new method for dialkyl peroxides synthesis in ionic liquids as solvents", GREEN CHEMISTRY, Bd. 8 2006, Seiten 292-295, XP002613529, Gefunden im Internet: URL:http://www.rsc.org/delivery/_ArticleLinking/DisplayArticleForFree.cfm?doi=b513863a&JournalCode=GC [gefunden am 2010-12-09]**
- **JAIN S L ET AL: "[Bmim]BF4-immobilized rhenium-catalyzed highly efficient oxygenation of aldimines to oxaziridines using solid peroxides as oxidants", JOURNAL OF ORGANOMETALLIC CHEMISTRY, Bd. 692, Nr. 14, 15. Juni 2007 (2007-06-15), Seiten 2930-2935, XP002613530, ELSEVIER NL DOI: 10.1016/J.JORGANCHEM.2007.03.004**
- **PANDEY S: "Analytical applications of room-temperature ionic liquids: A review of recent efforts", ANALYTICA CHIMICA ACTA - YOUNG ANALYTICAL FACULTY IN ASIA, Bd. 556, Nr. 1, 18. Januar 2006 (2006-01-18), Seiten 38-45, XP002613531, ELSEVIER NL DOI: 10.1016/J.ACA.2005.06.038**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **MIYAKE A ET AL: "Mixing hazard evaluation of organic peroxides with other chemicals", JOURNAL OF LOSS PREVENTION IN THE PROCESS INDUSTRIES - SELECTED PAPERS PRESENTED AT THE INTERNATIONAL CONFERENCE ON BHOPAL GAS TRAGEDY AND ITS EFFECTS ON PROCESS SAFETY, Bd. 18, Nr. 4-6, Juli 2005 (2005-07), Seiten 380-383, XP002613532, ELSEVIER LTD GB DOI: 10.1016/J.JLP.2005.06.021**
- **SINGH R P ET AL: "Energetic nitrogen-rich salts and ionic liquids", ANGEWANDTE CHEMIE - INTERNATIONAL EDITION, Bd. 45, Nr. 22, 26. Mai 2006 (2006-05-26), Seiten 3584-3601, XP002613533, WILEY-VCH VERLAG DE DOI: 10.1002/ANIE.200504236**

## Beschreibung

Die Erfindung betrifft die Verwendung einer neutralen ionischen Flüssigkeit zum Stabilisieren von peroxidischen Sprengstoffen und zum Herstellen stabiler Lösungen von peroxidischen Sprengstoffen. Die Erfindung betrifft weiterhin stabile Zusammensetzungen umfassend eine Lösung einer neutralen ionischen Flüssigkeit mit einer detektierbaren Menge eines peroxidischen Sprengstoffes, deren Herstellung und deren Verwendung als Duftprobe.

## Hintergrund der Erfindung

Peroxidische Sprengstoffe wie das TATP oder HMTD zählen zu den so genannten Selbstelaboraten, die leicht in großer Menge aus Haushaltschemikalien hergestellt werden können. Die hohe Brisanz und damit schlechte Handhabbarkeit selbst von kleinen Mengen dieses Stoffes ist für die Probenbereitstellung des Echtstoffes eine sicherheitstechnische Herausforderung. In lipophilen ionischen Flüssigkeiten gelöst können die peroxidischen Sprengstoffe wie das TATP sicher gehandhabt werden. Die nahezu konstante Abdampfrate des peroxidischen Sprengstoffes kann genutzt werden um das Duftprofil eines größeren Sprengsatzes zu simulieren. Die Verwendung zu Kalibrierungszwecken ist ebenfalls denkbar. Der nicht messbare Dampfdruck der ionischen Flüssigkeit beeinflusst die Probenqualität nicht. Das Lösen des peroxidischen Sprengstoffes in Kombination mit einer reduktiv wirkenden Komponente in der ionischen Flüssigkeit deaktiviert den Sprengstoff dauerhaft und kann zur Entschärfung genutzt werden.

Das Training von Spürhunden und die Kalibrierung von Messgeräten müssen mit dem Echtstoff erfolgen. Ersatzstoffe können deshalb nicht zum Einsatz kommen. Das Triacetontriperoxid (TATP) kann bei der Synthese als TATP-Hydrat erhalten werden, das leichter zu handhaben ist und auch eine geringfügig geringere Brisanz aufweist. Wohingegen das reine TATP aufgrund der Reib- und Stoßempfindlichkeit sowie der Zündung durch Elektrostatik praktisch eine Verwendung ausschließt (R. Meyer et al., in Explosives, 6th Ed. Wiley-VCH, Weinheim (2007) und J.C. Oxley et al., Propellants, Explosives, Pyrotechnics 30:127 (2005)). Zur sicheren Generierung kleiner Sprengstoffspuren können so genannte Echtstoff-Mikromengen-Prüfkörper (EMPK) verwendet werden. Hier befindet sich das Sprengstoffmaterial (0.5-2.0 mg) auf einem Metallschaum (EMPK® Sicherheitstest: Bundesanstalt f. Materialforschung und -prüfung AZ: II.3/4726/04 und Bescheinigung Wehrwissenschaftliches Institut für Werk-, Explosiv- und Betriebsstoffe (WIWEB) Dok.-Nr.: 330/27454/04; Quelle: http://www.duelsner.de/seiten/deutsch/produkt.html). Allerdings zeigte sich bei Untersuchungen im Luftstrom, dass diese EMPKs aufgrund der Flüchtigkeit der peroxidischen Sprengstoffe sehr schnell entladen werden und deshalb nur wenige Minuten genutzt werden können. Besonders für eine Übung im freien Feld mit natürlichen Luftbewegungen ist dies problematisch. Darüber hinaus können nur sehr kleine TATP-Konzentrationen pro EMPK erreicht werden. Sprengstoffproben (Mikromengen) in Acetonitril gelöst gibt es als Standards von mehreren Anbietern. Für TATP ist das z.B. die Fa. AccuStandard (Quelle: http://www.accustandard.com; Katalognummer: M-8330-ADD24, 0.1 mg TATP in 1 ml $CH_3CN$ kosten US$ 75). Die darin enthaltenen Mengen sind sehr gering und die Beimengung an toxischem und flüchtigen Lösungsmittel sind enorm, so dass eine Anwendung für Kalibrierung von Gasanalyten mit biologischen oder elektronischen Nasen schwierig bzw. prohibitiv ist. Die Aktualität der TATP-Problematik lässt sich an der Vielzahl der publizierten Methoden zum Nachweis ablesen (Detection of Liquid Explosives and Flammable Agents in Connection with Terrorism, Eds: H. Schubert, A. Kuznetsov, NATO Science for Peace and Security Series, Springer, Dordrecht (2008); R. Schulte-Ladbeck et al., Anal. Bioanal. Chem. 386: 559 (2006); R. A. A. Munoz et al., Analyst 132:560 (2007); D. Lu et al., Analyst 131:1279 (2006); D. F. Laine et al., Anal. Chim. Acta 608:56 (2008); R. Schulte-Ladbeck et al., Analyst 127:1152 (2002); M. E. Germain und M. J. Knapp, Inorg. Chem. 47:9748 (2008); S. Malashikhin und N. S. Finney, J. Am. Chem. Soc. 130:12846 (2008); US Patent 6,767,717; E. Sella und D. Shabat, Chem. Commun. 5701 (2008)). Zur Validierung dieser Detektionsmöglichkeiten bedarf es einer sicher handhabbaren und leistungsfähigen TATP-Quelle.

Die hohe Brisanz von peroxidischen Sprengstoffen gestaltet die Analytik von aufgefundenen Substanzen äußerst gefährlich, da für eine zweifelsfreie Identifikation dieser pulverförmigen Feststoffe Material mechanisch entnommen werden muss. Getrocknete peroxidische Sprengstoffe wie TATP und im Besonderen HMTD können dabei gezündet werden. Üblicherweise werden derartige Substanzfunde mit Dieseltreibstoff überschüttet, nach einer gewissen Zeit kann der Sprengstoff gefahrlos mechanisch aufgesammelt werden. Aufgrund der Komplexität des aus mehreren tausend flüchtigen Komponenten bestehenden Dieselgemisches sind keine späteren Analysen mehr möglich und eine forensische Verwertung ist damit unmöglich. Die bereitstellung eines geeigneten Phlegmatisierungsmittels ist deshalb wünschenswert.

Ionische Flüssigkeiten sind innovative Lösungsmittel mit einem vernachlässigbaren Dampfdruck (Ionic Liquids in Synthesis, Eds: P. Wasserscheidt, T. Welton, WILEY-VCH, Weinheim (2003)) und sollten deshalb den olfaktorischen Eindruck von TATP und anderen peroxidischen Sprengstoffen nicht beeinflussen. Die Phlegmatisierung von Sprengstoffen zur besseren Verarbeitbarkeit - vor allem Nitroverbindungen - wurde vor kurzem beschrieben US 20080251169 A1). Phlegmatisierte Peroxidlösungen sind aus US 20060135822 A1 bekannt. Die bislang handhabbaren TATP-Proben haben aus Sicherheitsgründen nur einen sehr geringen Echtstoffgehalt. Da das TATP als kristallines Material dort

aufgebracht wird ist die Brisanz des Explosivstoffes nach wie vor vorhanden. Die Duft-Simulation eines realen Sprengkörpers aus TATP oder Substanzfund mit Verdacht auf TATP kann deshalb nur unzureichend abgebildet werden. Außerdem sind die EMPKs im Luftstrom sehr schnell von der kleinen Analytmenge entladen. Darüber hinaus zeichnen sich die bekannten Probenkörper durch einen hohen Preis aus (0.5 mg TATP ca. € 40,-). Für Testmessungen im Luftstrom werden ca. 10 Probenkörper benötigt, die innerhalb einigen Minuten vom TATP befreit werden.

**Kurzbeschreibung der Erfindung**

Es wurde nun gefunden, dass durch Lösen von peroxidischen Sprengstoffen wie TATP oder TATP-Hydrat in speziellen ionischen Lösungsmitteln wie sie aus der WO 04/035018 bekannt sind, sich eine stabile und leicht handhabbare Form des Explosivstoffes TATP ergibt. Die dramatisch reduzierte mechanische und thermische Empfindlichkeit dieser Lösung erlaubt die einfache Handhabung in konventionellen Laboratorien mit der üblichen Ausrüstung. Die inzwischen untersuchten ionischen Flüssigkeiten zeigen keine messbare Reib- und Schlagempfindlichkeit mehr. Auch kann eine Phlegmatisierung unter gleichzeitiger Option zur Forensik durch die ionischen Flüssigkeiten erreicht werden. Lösungen von ionischen Flüssigkeiten in leicht flüchtigen Lösungsmitteln, wie z.B. Dichlormethan, können auf die Sprengstoffe getropft werden. Die Sprengstoffe werden zügig und gut benetzt. Aufgrund der Leitfähigkeit der ionischen Flüssigkeit werden auch elektrostatische Zündungen vermieden. Das erzeugte Gemenge zeigt keine Reib- und Schlagempfindlichkeit mehr. Die Erfindung betrifft somit

(1) die Verwendung einer neutralen ionischen Flüssigkeit (nachfolgend kurz "IF") zum Stabilisieren von peroxidischen Sprengstoffen (Phlegmatisierung) und zum Herstellen stabiler Lösungen von peroxidischen Sprengstoffen;
(2) eine stabile Zusammensetzung umfassend eine Lösung einer neutralen ionischen Flüssigkeit (IF) mit einer detektierbaren Menge eines peroxidischen Sprengstoffs;
(3) die Herstellung der Zusammensetzung von (2), umfassend das Auflösen des peroxidischen Sprengstoffs in der neutralen ionischen Flüssigkeit; und
(4) die Verwendung der Zusammensetzung von (2) als Duftquelle, insbesondere für Testmessungen, für die Kalibrierung Detektoren und zum Training von Sprengstoffspürhunden oder anderen biologischen Nachweistechniken; .

**Kurzbeschreibung der Figuren**

Fig. 1: DSC-Messung einer Ionischen Flüssigkeit (IF) mit und ohne TATP
Fig. 2: TGA-Messungen einer Ionischen Flüssigkeit (IF) mit und ohne TATP

**Detaillierte Beschreibung der Erfindung**

In der Verwendung gemäß Aspekt (1) der Erfindung und in der Zusammensetzung gemäß Aspekt (2) der Erfindung sind die lipophile ionische Flüssigkeiten von besonderer Bedeutung, da diese wenig Wasser aus der Umgebung aufnehmen. Da die Eigenschaften der ionischen Flüssigkeiten sowohl durch die Kationen als auch die Anionen bestimmt wird, können beide Seiten variiert werden. Vorzugsweise werden neutrale ionische Flüssigkeiten mit einer geringen Viskosität eingesetzt. Als lipophile Anionen eignen sich unter anderem Tetrafluoroborate, Triflitimide, Perfluoralkylsulfate, Alkylsulfonate, Arylsulfonate, Perfluoralkylsulfonate, bis-Perfluoralkylsulfonimide, Acetate, Alkylcarboxylate, Isocyanate, Isothiocyanate, Thiosulfate, Halogenide (einschließlich Iodide, Bromide, Chloride und Fluoride), Borate, Phosphate, Nitrate und Perchlorate, wobei Tetrafluorborate und Triflitimide besonders geeignet sind. Geeignete Kationen sind *N*-alkylsubstituierten Stickstoffheterozyklen, wie *N*-Alkylpyridinium-, *N*-Alkylpyrazinium-, *N*-Alkylpyridazinium-, *N*-Alkylpyrimidinium- und bis-*N*-Alkylimidazoliumionen, quatären Ammonium- und Phosphoniumionen, wobei *N,N*-Dialkylimidazolium- und *N*-Alkylpyridiniumionen besonders bevorzugt sind. Besonders bevorzugt sind dabei 1-Ethyl-3-methylimidazolium-bis(trifluormethansulfonimid), 1-Butyl-3-methylimidazolium-bis(trifluormethansulfonimid), 1-Hexyl-3-methylimidazolium-bis(trifluormethansulfonimid), 1-Ethyl-3-methylimidazolium-tetrafluoroborat, 1-Hexyl-3-methylimidazolium-tetrafluoroborat, 1-Octyl-3-methylimidazolium-tetrafluoroborat, 1-Decyl-3-methylimidazolium-tetrafluoroborat, *N*-Hexylpyridinium-tetrafluoroborat, *N*-Hexylpyridinium-bis(trifluomethansulfonimid), *N*-Butyl-3-methylpyridinium-tetrafluoroborat und *N*-Butyl-4-methylpyridinium-tetrafluoroborat.

Aufgrund der unpolaren Natur des TATP-Moleküls sind lipophile ionische Flüssigkeiten begünstigt. Diesen Trend kann man an der Löslichkeit von TATP in den 1-Alkyl-3-methylimidazoliumtetrafluoroboraten gut ablesen. Es ist daher bevorzugt, dass wenigstens ein Alkylrest des *N*,*N*-Dialkylimidazoliumions bzw. der *N*-Alkylrest des *N*-Alkylpyridiniums ein $C_6$ bis $C_{16}$-Alkylrest ist. Es können auch Gemische der genannten ionischen Flüssigkeit eingesetzt werden.

Peroxidische Sprengstoffe im Sinne der vorliegenden Erfindung sind dabei zyklische Peroxide wie Triacetontriperoxid (TATP), Hexamethylentriperoxiddiamin (HMTD), Diacetonperoxid usw. mit den Strukturen (I) bis (III)

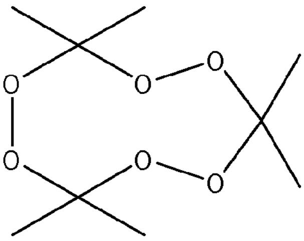

(I)

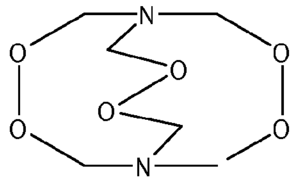

(II)

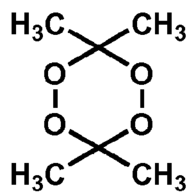

(III)

Diacylperoxide der nachfolgenden Formel (iV), worin R ein geradkettiger, verzweigter oder zyklischer, gesättigter $C_{1-5}$-Alkylrest oder ein mono oder polyzyklischer Arylrest ist, wobei die Alkyl- und Arylreste optional mit einem oder mehreren Resten ausgewählt aus Halogen, Nitro, Hydroxy und Oxo substituiert sein können, und wobei Diacetylperoxid und Bisbenzoylperoxid mit den Strukturen IVa und IVb besonders bevorzugt sind

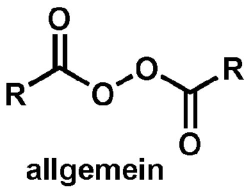

**allgemein**

(IV)

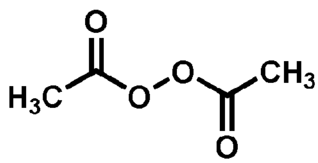

(IVa)

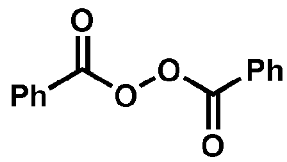

(IVb)

und sonstige leicht herstellbare Peroxide wie Bis(1-hydroxycyclohexyl)peroxid mit der nachfolgenden Formel V

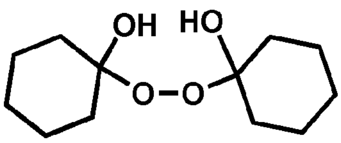

(V).

In einer Ausführungsform der Verwendung gemäß Aspekt (1) der Erfindung wird die IF zum Stabilisieren von peroxidischen Sprengstoffen (Phlegmatisierung) verwendet. Hierbei ist es bevorzugt, dass die IF als Gemisch mit einem flüchtigen Lösungsmittel eingesetzt wird. Geeignete flüchtige Lösungsmittel sind dabei schwer entzündliche, eine nachfolgende Analytik erlaubende Lösungsmittel einschließlich Dichlormethan, Chloroform, Acetonitril Essigester, wobei Dichlormethan besonders bevorzugt ist. Der Gehalt der IF in dem flüchtigen Lösungsmittel beträgt dabei 1 bis 25 Gew.-%, vorzugsweise 5 bis 15 Gew.-%.

In einer weiteren Ausführungsform der Verwendung gemäß Aspekt (1) der Erfindung wird die IF zum Herstellen einer stabilen Lösung von peroxidischen Sprengstoffen verwendet wird, wobei die stabile Lösung vorzugsweise als Duftquelle, insbesondere für Testmessungen für die Kalibrierung Detektoren und zum Training von Sprengstoffspürhunden oder anderen biologischen Nachweistechniken eingesetzt wird.

In der Zusammensetzung gemäß Aspekt (2) der Erfindung ist der Gehalt an peroxidischem Sprengstoff 0,1 bis 10 Gew.-%, vorzugsweise 0,5 bis 5 Gew.-% und besonders bevorzugt etwa 2 Gew.-% der Zusammensetzung beträgt (Rest IF).

Die Zusammensetzung gemäß Anspruch (2) kann neben IF und peroxidischem Sprengstoff weiterhin noch funktionelle Verbindungen wie Farbstoffe oder alternative Geruchsstoffe enthalten, die ihre Identifizierung erleichtern. In einer bevorzugten Ausführungsform besteht die Zusammensetzung ausschließlich aus IF und peroxidischem Sprengstoff.

Die Herstellung der Zusammensetzung gemäß Aspekt (3) der Erfindung erfolgt durch Zugabe eines Peroxids zu der ionischen Flüssigkeit und langsames Auflösen, das gegebenenfalls durch vorsichtiges Rühren unterstützt werden kann.

Zum einen können die peroxidischen Sprengstofflösungen in ionischen Flüssigkeiten Duftquellen mit einer sehr

hohen und lang anhaltenden Intensität gewährleisten, da in den Proben eine relativ große TATP Menge vorhanden ist. Die sichere Handhabung ist möglich, da TATP nicht kristallin vorliegt. Es wird eine ausgesprochene thermische und mechanische Stabilität beobachtet. Der nicht messbare Dampfdruck der ionischen Flüssigkeiten verfälscht das Duftprofil des TATPs nicht nennenswert. Haupteinsatz wird die sichere Duftsimulation einer größeren TATP-Sprengstoffmenge sein.

Zum anderen kann durch geringfügige Modifikation der ionischen Flüssigkeit ein aktives Phlegmatisierungsmittel zur Entschärfung von TATP-Mengen bereitgestellt werden

- sichere Handhabung von peroxidischen Sprengstoffen in ionischen Flüssigkeiten
- Erzeugung von Duftproben peroxidischer Sprengstoffe durch Lösen in ionischen Flüssigkeiten
- Erzeugung von Probenkörpern mit intensiver Duftsignatur
- Simulation von großen IED durch Verdampfung von Echtstoffen aus ionischen Flüssigkeiten
- Phlegmatisierender Abbau der peroxidischen Sprengstoffe durch reduktiv wirkende ionische Flüssigkeiten

Die Zusammensetzung gemäß Aspekt (2) der Erfindung bietet eine starke Sprengstoff (TATP)-Duftsignatur, Simulation realer Szenarien, Sprengstoff (TATP)-Entschärfung, Echtstoff-Prüfkörper, Sprengstoff, Peroxide, Ionische Flüssigkeit, Duftkörper, sichere Handhabung. Weiterhin ist sie einsetzbar für Testmessungen und Kalibrierung von Sprengstoff (TATP)-Detektoren sowie Sprengstoff (TATP)-Nachweissystemen unter realen Szenarien einer terroristischen Gefahr (Bombe auf TATP-Basis). Training von Sprengstoffspürhunden oder anderen biologischen Nachweistechniken.

Die Erfindung wird anhand der folgenden Beispiele näher erläutert.

**Beispiele**

Herstellung einer mit Peroxid/TATP gesättigten ionischen Flüssigkeit: 20 bis 50 ml der ionischen Flüssigkeit wird in einen 250 ml Kolben mit einem Magnetrührerkern gegeben. Vorzugsweise hat der Kolben eine weite Öffnung, um pulverförmiges Material leicht zugeben zu können, und der Boden sollte konisch geformt sein, um eine Dekantierung der Flüssigkeit nach der Auflösung zu erleichtern. Es muss beachtet werden, dass der Rührkern und der Magnetrührer genügend starken Magnetfelder besitzen, um auch zähflüssige Suspensionen zuverlässig mit hoher Drehzahl mischen zu können. Der Kolben wird auf einen Magnetrührer mit Stativ nahe an den Magneten gespannt. Der Rührer wird auf eine möglichst hohe Drehzahl eingestellt, so dass der Rührkern der Drehung noch zuverlässig folgen kann. In der Praxis beträgt die Drehzahl zwischen 700 und 1000 U/min. 500 mg des Peroxides/Triacetonperoxid-Hydrat (TATP) wird zugegeben, und der Kolben wird gasdicht verschlossen. Der Schliffstopfen wird mit Hochvakuumfett sorgfältig abgedichtet und zugeklemmt, damit kein Peroxid während des Auflösens entweichen kann. Das Gemisch wird so lange gerührt, bis das Peroxid vollständig aufgelöst ist. Dies dauert i.A. 8 bis 12 h. Falls das Peroxid nicht vollständig aufgelöst wurde, wird noch mindestens 48 h weiter gemischt, um sicherzustellen, dass die Lösung gesättigt ist. Falls das Peroxid vollständig gelöst wurde, wird ein zusätzlicher 500 mg Ansatz TATP zugegeben und wieder entweder bis vollständiger Auflösung oder 12 h gemischt. Dieser Vorgang wird so oft wiederholt, bis ungelöstes Peroxid in der Lösung verbleibt. Danach wird noch 48 h weiter gerührt.

Wenn die Lösungen gesättigt sind, wird der Rührer ausgeschaltet, und die Lösungen werden für mindestens 24 h stehen gelassen. Dabei sedimentiert das überschüssige Peroxid entweder auf der Oberfläche oder auf dem Boden - je nach Dichte der Flüssigkeit. Die klare Flüssigkeit wird mit einer Pipette sehr langsam abgesaugt und ins dichte Gebinde abgefüllt. Die Peroxidrückstände im Auflösungskolben werden mit 5 % Natriumdithionit in einem Aceton-Wassergemisch (70:30, Gewichtsanteile) entsorgt.

Es ist zu beachten, dass eine Filtrierung mit den zähflüssigen ionischen Flüssigkeiten ohne große Materialverluste nicht möglich ist. Daher muss das überschüssige Peroxid durch Dekantieren oder Zentrifugieren getrennt werden. Es bleiben dabei noch geringe Mengen von sehr kleinen Kristallen in der Flüssigkeit zurück. Dieser sehr kleine Überschuss stellt sicher, dass die TATP-Lösungen gesättigt bleiben, auch wenn kleine Mengen Acetonperoxid z.B. durch die Gebindedichtungen entweichen. Die Kristallreste stellen auch keine Gefahr durch erhöhte Empfindlichkeit dar, weil sie nur in so kleinen Mengen vorhanden sind. Die Eigenschaften der TATP-haltigen ionischen Flüssigkeiten sind in der nachfolgenden Tabelle 1 zusammengefasst.

Tab. 1: Übersicht über die Löslichkeit von TATP in einigen Ionischen Flüssigkeiten (IF)

| Ionische Flüssigkeit (IF) | M (IF) [g/mol] | rel.TATP-Integral | C[TATP] (w%) |
|---|---|---|---|
| 1-Ethyl-3-methylimidazolium-bis(trifluor-methansulfonimid) | 391,31 | 0,12 | 0,38 |
| 1-Butyl-3-methylimidazolium-bis(trifluor-methansulfonimid) | 419,36 | 0,32 | 0,94 |

(fortgesetzt)

| Ionische Flüssigkeit (IF) | M (IF) [g/mol] | rel.TATP-Integral | C[TATP] (w%) |
|---|---|---|---|
| 1-Hexyl-3-methylimidazolium-bis(trifluor-methansulfonimid) | 447,42 | 0,31 | 0,86 |
| 1-Ethyl-3-methylimidazolium-tetrafluoroborat | 197,97 | 0,02 | 0,12 |
| 1-Hexyl-3-methylimidazolium-tetrafluoroborat | 254,08 | 0,27 | 1,31 |
| 1-Octyl-3-methylimidazolium-tetrafluoroborat | 282,13 | 0,49 | 2,14 |
| 1-Hexyl-3-methylimidazolium-iodid | 294,18 | 0,09 | 0,38 |
| 1-Decyl-3-methylimidazoilium-tetrafluoroborat | 310,18 | 0,32 | 1,27 |
| N-Hexylpyridinium-tetrafluoroborat | 251,07 | 0,09 | 0,44 |
| N-Hexylpyridinium-bis(trifluomethansulfonimid) | 444,41 | 0,33 | 0,92 |
| N-Butyl-3-methylpyridinium-tetrafluoroborat | 237,05 | 0,06 | 0,31 |
| N-Butyl-4-methylpyridinium-tetrafluoroborat | 237,05 | 0,00 | 0,00 |

Bestimmung des TATP-Gehaltes *via* NMR-Sepktroskopie

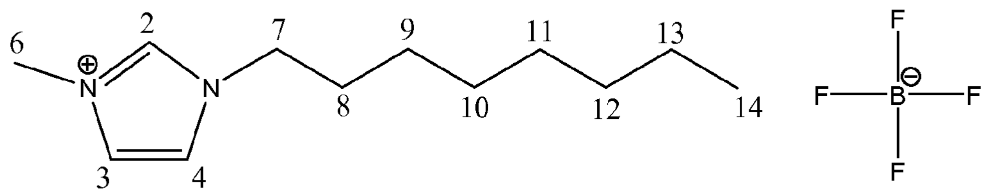

*1*-Octyl-3-methylimidazoliumtetrafluoroborat
$^1$H-NMR (400 MHz, DMSO-*d6*): 5 = 0.85 (t, 3H, H 14, $^3$J = 8.0 Hz), 1.25 (m, 10H, H 9-13), 1.36 (s, 0.49H, $CH_3$ TATP), 1.79 (m, 2H, H 8), 3.85 (s, 3H, H 6), 4.15 (t, 2H, H 7, $^3$J = 8.0 H), 7.70 (m, 2H, H3-4), 9.04(s, 1H, H 2).
Zur Konzentrationsbestimmung wird das TATP-Signal bei 1.36 ppm mit dem Signal der Methylgruppe des Imidazols bei 3.85 ppm in Relation gesetzt.

$$c\ (\text{Gew\% TATP}) = [M\ (\text{TATP}) \bullet (\text{TATP Signal}/6) \bullet 100] / M\ (\text{IF}) \qquad (1),$$

wobei M (TATP) = 222,2 g/mol beträgt.

Bestimmung der thermischen Stabilität: In den in Figur 1 gezeigten DSC Messungen ist zu sehen, dass die reine Ionische Flüssigkeit bis mindestens 250 °C stabil bleibt. Die mit TATP versetzte Ionische Flüssigkeit zeigt im Intervall von 165 °C bis 205 °C eine exotherme Reaktion, mit einem Zersetzungsmaximum bei 190 °C. Die Zersetzung erstreckt sich über einen Temperaturbereich von ungefähr 40 °C, was für eine langsame, nicht explosive, Zersetzung spricht. Der steigende Energiebedarf zwischen 25 °C und 100 °C liegt an gelöstem Wasser, das erst verdampft wird. Dieses ist leicht an der mit Wasser dotierten Ionischen Flüssigkeit zu sehen. Dies wird durch einen Kontrollversuch mit wassernasser ionischer Flüssigkeit belegt.
Das TATP in ionischer Flüssigkeit ist eine thermisch handhabbare, nicht explosive und sichere Darreichungsform für diesen Sprengstoff.

Bestimmung der Abdampfraten: Die in Figur 2 zusammengefassten TGA Messungen zeigen, dass über einen langen Zeitraum das TATP und das aufgrund des ionischen Charakters aufgenommene Wasser desorbiert werden. Durch die langsame Abdampfrate sollte die mit TATP dotierte ionische Flüssigkeit als Übungsquelle für verschiedene Nachweisverfahren geeignet sein. Der große lineare Bereich der TATP-Probe verspricht im Luftstrom eine lange und zuverlässige Einsatzzeit.

Vorschrift zur Sättigung der IFs mit HMTD: 20 bis 50 ml der ionischen Flüssigkeit wird in einen 250 ml Kolben mit einem Magnetrührkern gegeben. Vorzugsweise hat der Kolben eine weite Öffnung, um pulverförmiges Material leicht zugeben zu können, und der Boden sollte konisch geformt sein, um eine Dekantierung der Flüssigkeit nach der Auflösung

zu erleichtern. Mittels eines Wasserbades wird auf 30 °C thermostatisiert. Es muss beachtet werden, dass der Rührkern und der Magnetrührer genügend starke Magnetfelder besitzen, um auch zähflüssige Suspensionen zuverlässig mit hoher Drehzahl mischen zu können. Der Kolben wird auf einen Magnetrührer mit Stativ nahe an den Magneten gespannt. Der Rührer wird auf eine möglichst hohe Drehzahl eingestellt, so dass der Rührkern der Drehung noch zuverlässig folgen kann. In der Praxis beträgt die Drehzahl zwischen 700 und 1000 Umdrehungen pro Minute. 500 mg von HMTD werden zugegeben, und der Kolben wird gasdicht verschlossen. Der Schliffstopfen wird mit Hochvakuumfett sorgfältig abgedichtet und zugeklemmt, damit kein Peroxid während des Auflösens entweichen kann. HMTD-Zugaben werden solange in 24 h-Abständen wiederholt, bis sich kein HMTD mehr löst. Die Suspension wird danach noch eine Woche nachgerührt, bevor analog zur TATP-Sättigung dekantiert wird.
Der Gehalt an HMTD wird NMR-spektroskopisch bestimmt.

Beispielhafte Bestimmung des HMTD-Gehaltes

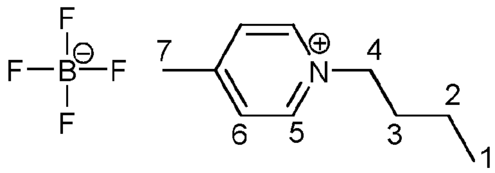

*N*-Butyl-4-methylpyridinium-tetrafluoroborat: $^1$H-NMR (400 MHz, DMSO-*d*6): $\delta$ = 0.88 (t, 3H, H 1, $^3J_{1,2}$ = 8.0 Hz), 1.25 (tq, 2H, H 2, $^3J_{2,1}$ = 8.0 Hz, $^3J_{2,3}$ = 8.0 Hz), 1.87 (tt, 2H, H 3, $^3J_{3,2}$ = 8.0 Hz, $^3J_{3,4}$ = 6.0 Hz), 2.60 (s,3H, H 7), 4.52 (t, 2H, H 4, $^3J_{4,3}$ = 6.0 Hz), 4.65 (d, 0.35H, $H_A$ HMTD, $^3J_{HA,HB}$ = 14.0 Hz), 4.77 (d, 0.35H, $H_B$ HMTD, $^3J_{HB,HA}$ = 14.0 Hz), 7.95 (d, 2H, H 6, $^3J_{6,5}$ = 4.0 Hz), 8.86 (d, 2H, H 5, $^3J_{5,6}$ = 8.0 Hz).

Zur Konzentrationsbestimmung von HMTD wird die Summe der Integrale bei 4.65 ppm und 4.77 ppm (entspricht 12 Protonen) mit dem Signal der Methylgruppe der Alkylkette bei 0.88 ppm (entspricht 3 Protonen) in Relation gesetzt. Zur Vereinfachung werden schon während der Auswertung die Integrale so eingepasst, dass für das Integral der IF-Methylprotonen (0.88 ppm) ein Wert von 3 erhalten wird.

$$c(HMTD) = \frac{HMTD - Signal/12}{IL - Signal/3} * \frac{M(HMTD)}{M(IF)}$$

Wegen der Normierung auf die Methyl-Protonen der IF wird der linke untere Term 1. Für die Darstellung in % wird mit 100 multipliziert.

$$c\ (HMTD)=[M\ (HMDT) \cdot (HMTD\ Signal/12) \cdot 100]\ /\ M\ (IF) \qquad (1),$$

wobei M (HMTD) = 208,10 g/mol beträgt.

Tabelle 2: Übersicht über die Löslichkeit von HMTD in ionischen Flüssigkeiten (IF)

| Ionische Flüssigkeit (IF) | M (IF) [g/mol] | rel. H MTD-Integral[a] | c[HMTD] (w%) |
|---|---|---|---|
| 1-Ethyl-3-methylimidazolium-bis(trifluormethansulfon)imid | 391,31 | 0,54 | 2,39 |
| 1-Butyl-1-methylpyrolidinium-bis(trifluormethansulfon) imid | 422,41 | 0,55 | 2,26 |
| *N*-Hexylpyridinium-bis(trifluormethansulfon)imid | 444,41 | 0,54 | 2,11 |
| *N*-Butyl-3-methylpyridinium-tetrafluoroborat | 237,05 | 0,23 | 1,68 |
| *N*-Butyl-3-methylpyridinium-tetrafluoroborat | 237,05 | 0,17 | 1,24 |
| *N*-Butyl-4-methylpyridinium-tetrafluoroborat | 237,05 | 0,70 | 5,12 |
| *N*-Hexylpyridinium-tetrafluoroborat | 251,07 | 0,04 | 0,28 |

(fortgesetzt)

| Ionische Flüssigkeit (IF) | M (IF) [g/mol] | rel. H MTD-Integral[a] | c[HMTD] (w%) |
|---|---|---|---|
| 1-Hexyl-3-methylimidazolium-bis(trifluormethansulfon) imid | 447,42 | 1,17 | 4,53 |
| 1-Ethyl-3-methylimidazolium-diethylphosphat | 264,26 | 0,14 | 0,92 |
| 1-Ethyl-3-methylimidazolium-methansulfonat | 206,26 | 0,48 | 4,04 |
| 1-Butyl-1-methylpyrrolidinium-trifluormethansulfonat | 291,34 | 0,17 | 1,01 |

[a] Zur Berechnung des relativen Signals werden im NMR die Integrale durch Normierung einer Methylgruppe der IL auf 3 eingestellt. Das relative Signal ist die Summe von beiden HMTD-Protonensignalen nach dieser Normierung und repräsentiert somit das Integral der 12 HMTD-Protonen pro Molekül IL (s.o.).

Phlegmatisierung peroxidischer Sprengstoffe

*Phlegmatisierungs-Lösung:* 10 % 1-Hexyl-3-methylimidazolium bis(trifluormethylsulfon)imid in Dichlormethan

Vor Phlegmatisierungsbehandlung:

Reibempfindlichkeit: HMTD 0,05 N - 100% Zündung; TATP 0,2 N - 100% Zündung.
Schlagempfindlichkeit: HMTD 0,2 J -100% Zündung, TATP 0,5 J - 100% Zündung.
Phlegmatisierungsversuche: 100 mg Sprengstoff werden mit 200 mg 10% Phlegmatisierungslösung angefeuchtet, 15 min trocknen gelassen und dann vermessen. Im Rahmen des Messbereichs konnten die beiden phlegmatisierten Sprengstoffe nicht mehr gezündet werden: >1 J Schlag- und >30 N Reibempfindlichkeit.

**Patentansprüche**

1. Verwendung einer ionischen Flüssigkeit (IF), die IF ausgewählt ist aus Tetrafluorborat-, Triflitimid-, Perfluoralkylsulfat-, Alkylsulfonat-, Arylsulfonat-, Perfluoralkylsulfonat-, bis-Perfluoralkylsulfonimid-, Acetat-, Alkylcarboxylat-, Isocyanat-, Isothiocyanat-, Thiosulfat-, Halogenid-, Borat-, Phosphat-, Nitrat- und Perchloratsalzen von *N*-alkylsubstituierten Stickstoffheterozyklen, quatären Ammonium und Phosphonium, zur Phlegmatisierung von peroxidischen Sprengstoffen und zum Reduzieren der mechanischen und thermischen Empfindlichkeit von peroxidischen Sprengstoffen.

2. Verwendung nach Anspruch 1, wobei die substituierten Stickstoffheterozyklen ausgewählt sind aus *N*-Alkylpyridinium, *N*-Alkylpyrazinium, *N*-Alkylpyridazinium, *N*-Alkylpyrimidinium und bis-*N*-Alkylimidazolium, wobei Tetrafluorborate und bis-Perfluoralkylsulfonimide von 1,3-bis-N-Alkylimidazolium und N-Alkylpyridinium besonders bevorzugt sind.

3. Verwendung nach Anspruch 2, wobei die IF ausgewählt ist aus 1-Ethyl-3-methylimidazolium-bis(trifluormethansulfonimid), 1-Butyl-3-methylimidazolium-bis(trifluormethansulfonimid), 1-Hexyl-3-methylimidazolium-bis(trifluormethan-sulfonimid), 1-Ethyl-3-methylimidazolium-tetrafluoroborat, 1-Hexyl-3-methyl-imidazolium-tetrafluoroborat, 1-Octyl-3-methylimidazolium-tetrafluoroborat, 1-Decyl-3-methylimidazolium-tetrafluoroborat, *N*-Hexylpyridinium-tetrafluoroborat, *N*-Hexylpyridinium-bis(trifluormethansulfonimid), *N*-Butyl-3-methylpyridinium-tetrafluoroborat und *N*-Butyl-4-methylpyridinium-tetrafluoroborat.

4. Verwendung nach einem oder mehreren der Ansprüche 1 bis 3, wobei der peroxidische Sprengstoff ausgewählt ist aus Triacetontriperoxid (TATP), Hexamethylentriperoxiddiamin (HMTD), Diacetonperoxid, Diacylperoxiden der Formel R(C=O)-OO-(C=O)R (worin R ein geradkettiger, verzweigter oder zyklischer, gesättigter $C_{1-5}$-Alkylrest oder ein mono oder polyzyklischer Arylrest ist, wobei die Alkyl- und Arylreste optional mit einem oder mehreren Resten ausgewählt aus Halogen, Nitro, Hydroxy, Oxo substituiert sein können) und sonstige leicht herstellbare Peroxide wie Bis(1-hydroxycyclohexyl)peroxid, und wobei TATP und HMTD besonders bevorzugt sind.

5. Verwendung nach einem oder mehreren der Ansprüche 1 bis 4, wobei die ionische Flüssigkeit (IF) zur Phlegmatisierung verwendet wird, und wobei vorzugsweise die ionische Flüssigkeit als Gemisch mit einem flüchtigen Lösungsmittel eingesetzt wird.

6. Verwendung nach Anspruch 5, wobei das flüchtige Lösungsmittel ein schwer entzündliches eine nachfolgende Analytik erlaubendes Lösungsmittel einschließlich Dichlormethan, Chloroform, Acetonitril und Essigester ist, wobei Dichlormethan besonders bevorzugt ist.

7. Verwendung nach einem oder mehreren der Ansprüche 1 bis 4, wobei die ionische Flüssigkeit (IF) zum Reduzieren der mechanischen und thermischen Empfindlichkeit von peroxidischen Sprengstoffen verwendet wird, wobei die Lösung mit reduzierter mechanischer und thermischer Empfindlichkeit vorzugsweise als Duftquelle, insbesondere für Testmessungen für die Kalibrierung Detektoren und zum Training von Sprengstoffspürhunden oder anderen biologischen Nachweistechniken eingesetzt wird

8. Zusammensetzung mit reduzierter mechanischer und thermischer Empfindlichkeit, umfassend eine Lösung wenigstens einer ionischen Flüssigkeit (IF), ausgewählt aus Tetrafluorborat-, Triflitimid-, Perfluoralkylsulfat-, Alkylsulfonat-, Arylsulfonat-, Perfluoralkylsulfonat-, bis-Perfluoralkylsulfonimid-, Acetat-, Alkylcarboxylat-, Isocyanat-, Isothiocyanat-, Thiosulfat-, Halogenid-, Borat-, Phosphat-, Nitrat- und Perchloratsalzen von *N*-alkylsubstituierten Stickstoffheterozyklen, quatären Ammonium und Phosphonium, mit einem Gehalt von 0,1 bis 10 Gew.-% eines peroxidischen Sprengstoffes.

9. Zusammensetzung nach Anspruch 8, wobei die *N*-alkylsubstituierten Stickstoffheterozyklen ausgewählt sind aus *N*-Alkylpyridinium, *N*-Alkylpyrazinium, *N*-Alkylpyridazinium, *N*-Alkylpyrimidinium und bis-*N*-Alkylimidazolium, wobei Tetrafluorborate und bis-Perfluoralkylsulfonimide von 1,3-bis-N-Alkylimidazolium und N-Alkylpyridinium besonders bevorzugt sind.

10. Zusammensetzung nach Anspruch 9, wobei die IF ausgewählt ist aus 1-Ethyl-3-methylimidazolium-bis(trifluormethansulfonimid), 1-Butyl-3-methylimidazolium-bis(trifluormethansulfonimid), 1-Hexyl-3-methylimidazolium-bis(trifluormethan-sulfonimid), 1-Ethyl-3-methylimidazolium-tetrafluoroborat, 1-Hexyl-3-methyl-imidazolium-tetrafluoroborat, 1-Octyl-3-methylimidazolium-tetrafluoroborat, 1-Decyl-3-methylimidazolium-tetrafluoroborat, *N*-Hexylpyridinium-tetrafluoroborat, *N*-Hexylpyridinium-bis(trifluormethansulfonimid), *N*-Butyl-3-methylpyridinium-tetrafluoroborat und *N*-Butyl-4-methylpyridinium-tetrafluoroborat.

11. Zusammensetzung nach einem oder mehreren der Ansprüche 8 bis 10, wobei der peroxidische Sprengstoff ausgewählt ist aus Triacetontriperoxid (TATP), Hexamethylentriperoxiddiamin (HMTD), Diacetonperoxid, Diacylperoxiden der Formel R(C=O)-OO-(C=O)R (worin R ein geradkettiger, verzweigter oder zyklischer, gesättigter $C_{1-5}$-Alkylrest oder ein mono oder polyzyklischer Arylrest ist, wobei die Alkyl- und Arylreste optional mit einem oder mehreren Resten ausgewählt aus Halogen, Nitro, Hydroxy, Oxo substituiert sein können) und sonstige leicht herstellbare Peroxide wie Bis(1-hydroxycyclohexyl)peroxid, und wobei TATP und HMTD besonders bevorzugt sind.

12. Zusammensetzung nach einem oder mehreren der Anspruche 8 bis 11, wobei der Gehalt an peroxidischem Sprengstoff 0,5 bis 5 Gew.-% und bevorzugt etwa 2 Gew.-% der Zusammensetzung beträgt.

13. Zusammensetzung nach einem der Ansprüche 8 bis 12, die ausschließlich aus IF und peroxidischem Sprengstoff besteht.

14. Zusammensetzung nach einem oder mehreren der Anspruche 8 bis 13, die eine Duftquelle ist.

15. Verfahren zur Herstellung der Zusammensetzung nach einem der Ansprüche 8 bis 14, umfassend das Auflösen des peroxidischen Sprengstoffs in der neutralen ionischen Flüssigkeit.

16. Verwendung der Zusammensetzung gemäß einem oder mehreren der Ansprüche 8 bis 14 als Duftquelle, insbesondere für Testmessungen für die Kalibrierung Detektoren und zum Training von Sprengstoffspürhunden oder anderen biologischen Nachweistechniken.

## Claims

1. Use of an ionic liquid (IL) selected from tetrafluoroborate, triflitimide, perfluoroalkylsulfate, alkylsulfonate, arylsulfonate, perfluoroalkylsulfonate, bis(perfluoroalkyl)sulfonimide, acetate, alkylcarboxylate, isocyanate, isothiocyanate, thiosulfate, halide, borate, phosphate, nitrate, and perchlorate salts of N-alkyl-substituted nitrogen heterocycles, quaternary ammonium and phosphonium, for phlegmatizing peroxidic explosives, and for reducing the mechanical

and thermal sensitivity of peroxidic explosives.

2. The use according to claim 1, wherein said substituted nitrogen heterocycles are selected from N-alkylpyridinium, N-alkylpyrazinium, N-alkylpyridazinium, N-alkylpyrimidinium and bis-N-alkylimidazolium, wherein tetrafluoroborates and bis(perfluoroalkyl)sulfonimides of 1,3-bis(N-alkyl)imidazolium and N-alkylpyridinium are particularly preferred.

3. The use according to claim 2, wherein said IL is selected from 1-ethyl-3-methylimidazolium bis(trifluoromethane) sulfonimide, 1-butyl-3-methylimidazolium bis(trifluoromethane)sulfonimide, 1-hexyl-3-methylimidazolium bis(trifluoromethane) sulfonimide, 1-ethyl-3-methylimidazolium tetrafluoroborate, 1-hexyl-3-methylimidazolium tetrafluoroborate, 1-octyl-3-methylimidazolium tetrafluoroborate, 1-decyl-3-methylimidazolium tetrafluoroborate, N-hexylpyridinium tetrafluoroborate, N-hexylpyridinium bis(trifluoromethane)sulfonimide, N-butyl-3-methylpyridinium tetrafluoroborate, and N-butyl-4-methylpyridinium tetrafluoroborate.

4. The use according to one or more of claims 1 to 3, wherein said peroxidic explosive is selected from triacetone triperoxide (TATP), hexamethylene triperoxide diamine (HMTD), diacetone peroxide, diacyl peroxides of the formula R(C=O)-OO-(C=O)R (wherein R is a straight-chain, a branched or a cyclic, saturated $C_{1-5}$-alkyl radical or a monocyclic or a polycyclic aryl radical, (wherein said alkyl and aryl radicals may optionally be substituted by one or more radicals selected from halogen, nitro, hydroxy, oxo), and other peroxides that can be easily produced, such as bis(1-hydroxycyclohexyl)peroxide, and wherein TATP and HMTD are particularly preferred.

5. The use according to one or more of claims 1 to 4, wherein said ionic liquid (IL) is used for phlegmatization, and wherein preferably said ionic liquid is employed in admixture with a volatile solvent.

6. The use according to claim 5, wherein said volatile solvent is a solvent that is not readily flammable and allows for subsequent analysis, including dichloromethane, chloroform, acetonitrile, and ethyl acetate, wherein dichloromethane is particularly preferred.

7. The use according to one or more of claims 1 to 4, wherein said ionic liquid (IL) is used for reducing the mechanical and thermal sensitivity of peroxidic explosives, wherein said solution having reduced mechanical and thermal sensitivity is preferably employed as a scent source, especially for test measurements for the calibration of detectors, and for training explosive detection dogs, or for other biological detection techniques.

8. A composition having reduced mechanical and thermal sensitivity, comprising a solution of at least one ionic liquid (IL) selected from tetrafluoroborate, triflitimide, perfluoroalkylsulfate, alkylsulfonate, arylsulfonate, perfluoroalkylsulfonate, bis(perfluoroalkyl)sulfonimide, acetate, alkylcarboxylate, isocyanate, isothiocyanate, thiosulfate, halide, borate, phosphate, nitrate, and perchlorate salts of N-alkyl-substituted nitrogen heterocycles, quaternary ammonium and phosphonium, with a content of from 0.1 to 10% by weight of a peroxidic explosive.

9. The composition according to claim 8, wherein said N-alkyl substituted nitrogen heterocycles are selected from N-alkylpyridinium, N-alkylpyrazinium, N-alkylpyridazinium, N-alkylpyrimidinium and bis-N-alkylimidazolium, wherein tetrafluoroborates and bis(perfluoroalkyl)sulfonimides of 1,3-bis(N-alkyl)imidazolium and N-alkylpyridinium are particularly preferred.

10. The composition according to claim 9, wherein said IL is selected from 1-ethyl-3-methylimidazolium bis(trifluoromethane) sulfonimide, 1-butyl-3-methylimidazolium bis(trifluoromethane)sulfonimide, 1-hexyl-3-methylimidazolium bis(trifluoromethane) sulfonimide, 1-ethyl-3-methylimidazolium tetrafluoroborate, 1-hexyl-3-methylimidazolium tetrafluoroborate, 1-octyl-3-methylimidazolium tetrafluoroborate, 1-decyl-3-methylimidazolium tetrafluoroborate, N-hexylpyridinium tetrafluoroborate, N-hexylpyridinium bis(trifluoromethane)sulfonimide, N-butyl-3-methylpyridinium tetrafluoroborate, and N-butyl-4-methylpyridinium tetrafluoroborate.

11. The composition according to one or more of claims 8 to 10, wherein said peroxidic explosive is selected from triacetone triperoxide (TATP), hexamethylene triperoxide diamine (HMTD), diacetone peroxide, diacyl peroxides of the formula R(C=O)-OO-(C=O)R (wherein R is a straight-chain, a branched or a cyclic, saturated $C_{1-5}$-alkyl radical or a monocyclic or a polycyclic aryl radical, wherein said alkyl and aryl radicals may optionally be substituted by one or more radicals selected from halogen, nitro, hydroxy, oxo), and other peroxides that can be easily produced, such as bis(1-hydroxycyclohexyl)peroxide, and wherein TATP and HMTD are particularly preferred.

12. The composition according to one or more of claims 8 to 11, wherein said content of peroxidic explosive is from 0.5

to 5% by weight, preferably about 2% by weight, of the composition.

**13.** The composition according to one or more of claims 8 to 12, which consists exclusively of the IL and the peroxidic explosive.

**14.** The composition according to one or more of claims 8 to 13, which is a scent source.

**15.** A process for producing the composition according to any of claims 8 to 14, comprising dissolving the peroxidic explosive in said neutral ionic liquid.

**16.** Use of the composition according to one or more of claims 8 to 14 as a scent source, especially for test measurements for the calibration of detectors, and for training explosive detection dogs, or for other biological detection techniques.

## Revendications

**1.** Utilisation d'un fluide ionique (FI), le FI étant sélectionné parmi les sels tétrafluoroborate, triflitimidure, perfluoroalkylsulfate, alkylsulfonate, arylsulfonate, perfluoroalkylsulfonate, bis-perfluoroalkylsulfonimidure, acétate, alkylcarboxylate, isocyanate, isothiocyanate, thiosulfate, halogénure, borate, phosphate, nitrate et perchlorate d'hétérocycles azotés N-alkylsubstitués, d'ammonium et de phosphonium quaternaires, pour la flegmatisation d'explosifs peroxydiques et pour la réduction de la sensibilité mécanique et thermique d'explosifs peroxydiques.

**2.** Utilisation selon la revendication 1, les hétérocycles azotés substitués étant sélectionnés parmi les groupes N-alkylpyridinium, N-alkylpyrazinium, N-alkylpyridazinium, N-alkylpyrimidinium et bis-N-alkylimidazolium, les tétrafluoroborates et les bis-perfluoroalkylsulfonimidures de 1,3-bis-N-alkylimidazolium et de N-alkylpyridinium étant particulièrement préférés.

**3.** Utilisation selon la revendication 2, le FI étant sélectionné parmi le bis(trifluorométhanesulfonimidure) de 1-éthyl-3-méthylimidazolium, le bis(trifluorométhanesulfonimidure) de 1-butyl-3-méthylimidazolium, le bis(trifluorométhanesulfonimidure) de 1-hexyl-3-méthylimidazolium, le tétrafluoroborate de 1-éthyl-3-méthylimidazolium, le tétrafluoroborate de 1-hexyl-3-méthylimidazolium, le tétrafluoroborate de 1-octyl-3-méthylimidazolium, le tétrafluoroborate de 1-décyl-3-méthylimidazolium, le tétrafluoroborate de N-hexylpyridinium, le bis(trifluorométhanesulfonimidure) de N-hexylpyridinium, le tétrafluoroborate de N-butyl-3-méthylpyridinium, et le tétrafluoroborate de N-butyl-4-méthylpyridinium.

**4.** Utilisation selon une ou plusieurs des revendications 1 à 3, l'explosif peroxydique étant sélectionné parmi le tripероxyde de triacétone (TATP), l'hexaméthylènetriperoxydiamine (HMTD), le peroxyde de diacétone, les peroxydes de diacyle de formule R(C=O)-OO-(C=O)R (où R est un radical alkyle en $C_{1-5}$ saturé linéaire, ramifié ou cyclique ou un radical aryle mono- ou polycyclique, où les radicaux alkyle ou aryle peuvent en option être substitués par un ou plusieurs radicaux sélectionnés parmi un halogène, un groupe nitro, hydroxy ou oxo) et d'autres peroxydes pouvant être facilement produits, comme le peroxyde de bis(1-hydroxycyclohexyle), et le TATP et le HMTD étant particulièrement préférés.

**5.** Utilisation selon une ou plusieurs des revendications 1 à 4, le fluide ionique (FI) étant utilisé pour la flegmatisation, et de préférence le fluide ionique étant utilisé en tant que mélange avec un solvant volatile.

**6.** Utilisation selon la revendication 5, le solvant volatile étant un solvant difficilement inflammable autorisant une analyse ultérieure et comprenant le dichlorométhane, le chloroforme, l'acétonitrile et l'acétate d'éthyle, le dichlorométhane étant particulièrement préféré.

**7.** Utilisation selon une ou plusieurs des revendications 1 à 4, le fluide ionique (FI) étant utilisé pour réduire la sensibilité mécanique et thermique des explosifs peroxydiques, la solution présentant une sensibilité mécanique et thermique réduite étant utilisée de préférence en tant que source odoriférante, en particulier pour des mesures d'essai destinées à l'étalonnage de détecteurs et à la formation de chiens renifleurs d'explosifs ou d'autres techniques de détection biologiques.

**8.** Composition présentant une sensibilité mécanique et thermique réduite, comprenant une solution d'au moins un fluide ionique (FI) sélectionné parmi les sels tétrafluoroborate, triflitimidure, perfluoroalkylsulfate, alkylsulfonate,

arylsulfonate, perfluoroalkylsulfonate, bis-perfluoroalkylsulfonimidure, acétate, alkylcarboxylate, isocyanate, isothiocyanate, thiosulfate, halogénure, borate, phosphate, nitrate et perchlorate d'hétérocycles azotés N-alkylsubstitués, d'ammonium et de phosphonium quaternaires , présentant une teneur de 0,1 à 10 % en poids d'un explosif peroxydique.

**9.** Composition selon la revendication 8, dans laquelle les hétérocycles azotés N-alkylsubstitués sont sélectionnés parmi les groupes N-alkylpyridinium, N-alkylpyrazinium, N-alkylpyridazinium, N-alkylpyrimidinium et bis-N-alkylimidazolium, les tétrafluoroborates et les bis-perfluoroalkylsulfonimidures de 1,3-bis-N-alkylimidazolium et de N-alkylpyridinium étant particulièrement préférés.

**10.** Composition selon la revendication 9, dans laquelle la FI est sélectionnée parmi le bis(trifluorométhanesulfonimidure) de 1-éthyl-3-méthylimidazolium, le bis(trifluorométhanesulfonimidure) de 1-butyl-3-méthylimidazolium, le bis(trifluorométhanesulfonimidure) de 1-hexyl-3-méthylimidazolium, le tétrafluoroborate de 1-éthyl-3-méthylimidazolium, le tétrafluoroborate de 1-hexyl-3-méthylimidazolium-, le tétrafluoroborate de 1-octyl-3-méthylimidazolium, le tétrafluoroborate de 1-décyl-3-méthylimidazolium, le tétrafluoroborate de N-hexylpyridinium, le bis(trifluorométhanesulfonimidure) de N-hexylpyridinium, le tétrafluoroborate de N-butyl-3-méthylpyridinium, et le tétrafluoroborate de N-butyl-4-méthylpyridinium.

**11.** Composition selon une ou plusieurs des revendications 8 à 10, dans laquelle l'explosif peroxydique est sélectionné parmi le triperoxyde de triacétone (TATP), l'hexaméthylènetriperoxydiamine (HMTD), le peroxyde de diacétone, les peroxydes de diacyle de formule R(C=O)-OO-(C=O)R (où R est un radical alkyle en $C_{1-5}$ saturé linéaire, ramifié ou cyclique ou un radical aryle mono- ou polycyclique, où les radicaux alkyle ou aryle peuvent en option être substitués par un ou plusieurs radicaux sélectionnés parmi un halogène, un groupe nitro, hydroxy ou oxo) et d'autres peroxydes pouvant être facilement produits, comme le peroxyde de bis(1-hydroxycyclohexyle), et où le TATP et le HMTD sont particulièrement préférés.

**12.** Composition selon une ou plusieurs des revendications 8 à 11, dans laquelle la teneur en explosif peroxydique est comprise entre 0,5 et 5 % en poids, et est de préférence égale à environ 2 % en poids de la composition.

**13.** Composition selon l'une des revendications 8 à 12, qui se compose exclusivement de FI et d'un explosif peroxydique.

**14.** Composition selon une ou plusieurs des revendications 8 à 13, qui est une source odoriférante.

**15.** Procédé de production de la composition selon l'une des revendications 8 à 14, comprenant la dissolution de l'explosif peroxydique dans le fluide ionique neutre.

**16.** Utilisation de la composition selon une ou plusieurs des revendications 8 à 14 en tant que source odoriférante, en particulier pour des mesures d'essai destinées à l'étalonnage de détecteurs et à la formation de chiens renifleurs d'explosifs ou d'autres techniques de détection biologiques.

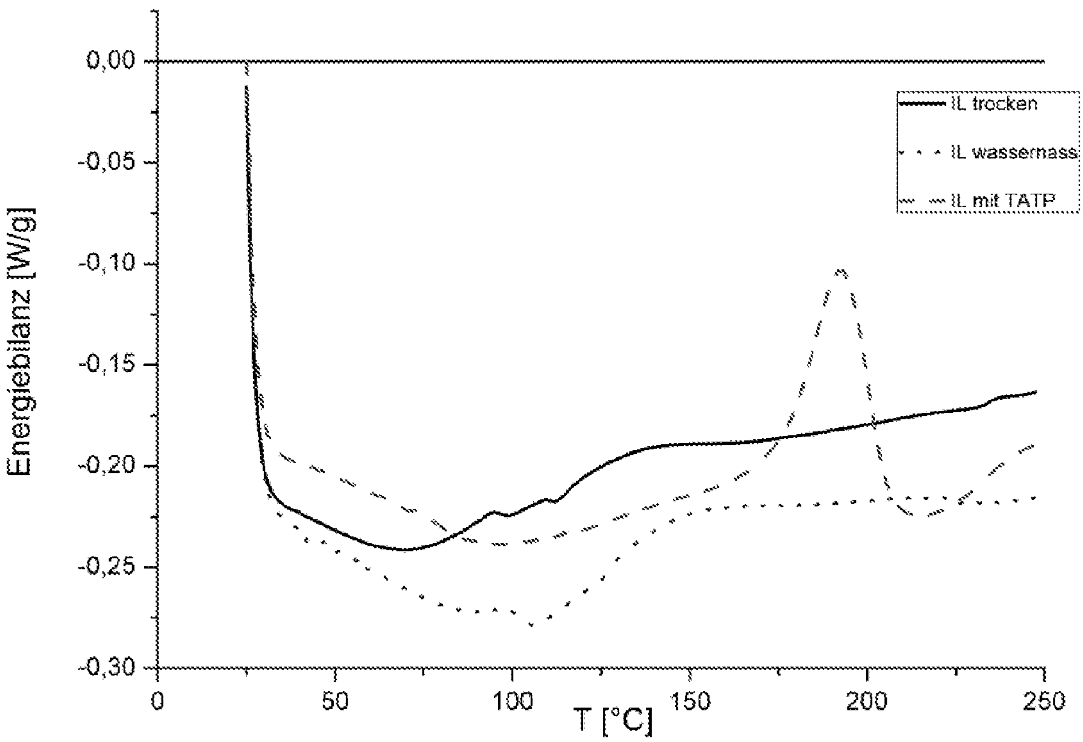
0,00
-0,05
-0,10
-0,15
-0,20
-0,25
-0,30
Energiebilanz [W/g]
0
50
100
150
200
250
T [°C]
IL trocken
IL wassernass
IL mit TATP

Fig. 1

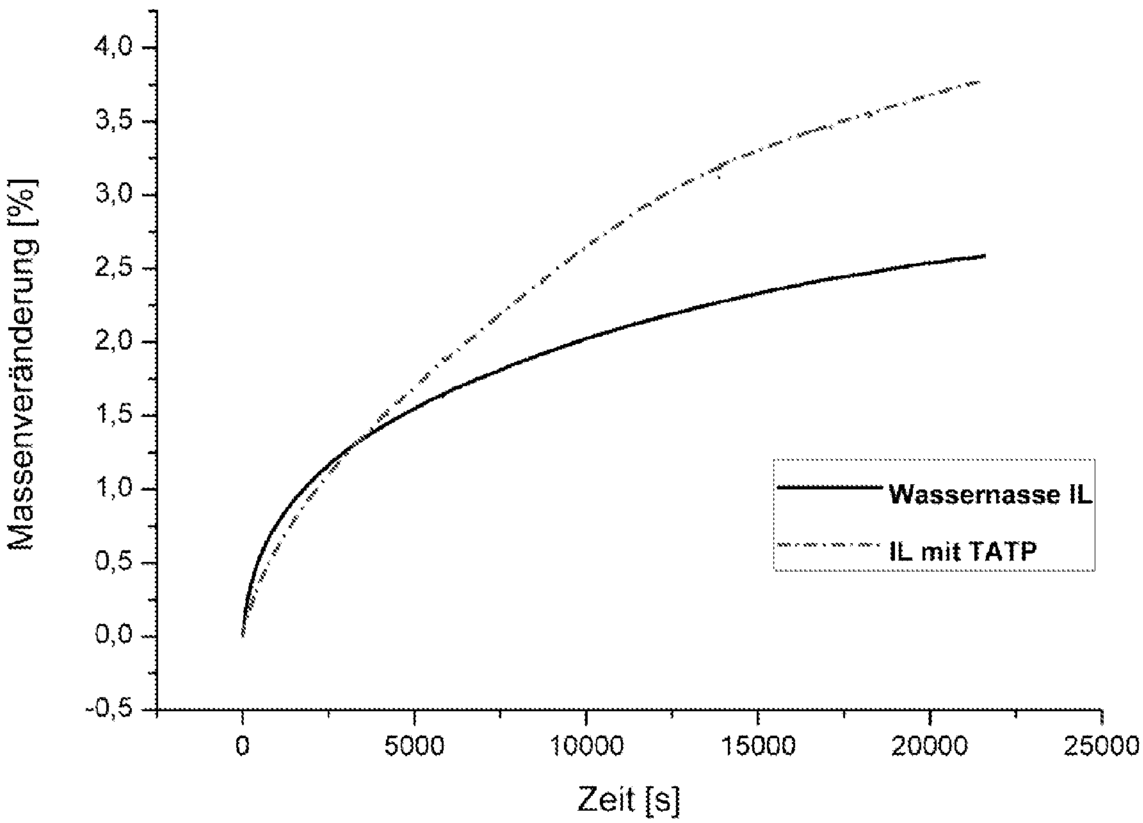
4,0
3,5
3,0
2,5
2,0
1,5
1,0
0,5
0,0
-0,5
Massenveränderung [%]
0
5000
10000
15000
20000
25000
Zeit [s]
Wassernasse IL
IL mit TATP

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6767717 B
- US 20080251169 A1
- US 20060135822 A1
- WO 04035018 A


### In der Beschreibung aufgeführte Nicht-Patentliteratur


- **R. MEYER et al.** Explosives. Wiley-VCH, 2007
- **J.C. OXLEY et al.** Propellants, Explosives. *Pyrotechnics,* 2005, vol. 30, 127
- NATO Science for Peace and Security Series. Springer, 2008
- **R. SCHULTE-LADBECK et al.** *Anal. Bioanal. Chem.,* 2006, vol. 386, 559
- **R. A. A. MUNOZ et al.** *Analyst,* 2007, vol. 132, 560
- **D. LU et al.** *Analyst,* 2006, vol. 131, 1279
- **D. F. LAINE et al.** *Anal. Chim. Acta,* 2008, vol. 608, 56
- **R. SCHULTE-LADBECK et al.** *Analyst,* 2002, vol. 127, 1152
- **M. E. GERMAIN ; M. J. KNAPP.** *Inorg. Chem.,* 2008, vol. 47, 9748
- **S. MALASHIKHIN ; N. S. FINNEY.** *J. Am. Chem. Soc.,* 2008, vol. 130, 12846
- **E. SELLA ; D. SHABAT.** *Chem. Commun.,* 2008, 5701
- Ionic Liquids in Synthesis. WILEY-VCH, 2003